# EUROPÄISCHE PATENTANMELDUNG

(11) **EP 0 551 644 A1**
(43) Veröffentlichungstag der Anmeldung: **21.07.1993**
(21) Anmeldenummer: 92121814.5
(22) Anmeldetag: 22.12.1992
(51) Int. Cl.: B01J 35/10, B01J 23/70, B01J 35/02, B01J 37/00

(54) **Katalysator-Formkörper**

(30) Priorität: 23.12.1991 DE 4142897
(71) Anmelder: SÜD-CHEMIE AG, D-80333 München (DE)
(72) Erfinder: Schneider, Michael, Dr., W-8012 Ottobrunn (DE); Kochloefl, Karl, Dr., W-8206 Bruckmühl/Heufeld (DE); Maletz, Gerd, Dr., W-8206 Bruckmühl (DE); Danner, Alfred, Dr., W-8201 Bad Feilnbach (DE)
(74) Vertreter: Reitzner, Bruno, Dr.

(57) **Zusammenfassung**

Beschrieben wird ein Katalysator-Formkörper, der im reduzierten Zustand ein Metall der Eisengruppe und/oder Kupfer auf einen Träger enthält, und der durch folgende Merkmale gekennzeichnet ist:
(a) Metallgehalt: 5-40 Gew.%, bezogen auf das Gesamtgewicht des Katalysators.
(b) Metall-Kristallitgröße ≦ 3nm
(c) Volumen der Poren mit einem Durchmesser von 7,5nm bis 15µm: 0,05 bis 0,9 ml/g Katalysator;
(d) BET-Oberfläche: 80-400 m²/g Katalysator;
(e) Bruchfestigkeit > 30 N (bezogen auf einen zylindrischen Formkörper mit einem Durchmesser von 1,5mm und einer Länge von 5mm);
(f) Schüttgewicht: 250-350g/l.

## Beschreibung

Die Erfindung betrifft poröse Katalysator-Formkörper, die im reduzierten Zustand ein Metall der Eisengruppe und/oder Kupfer auf einem Träger enthalten.

Metallkatalysatoren auf der Basis von Fe, Co, Ni und Cu finden bereits Anwendung bei Hydrierungen von Kohlenwasserstoffen, Methanisierungen und Oligomerisierungen. Wichtig für die Aktivität ist dabei eine hohe spezifische Metalloberfläche. Diese erreicht man u.a. durch hohe Metallbeladungen, die aber relativ große Metallkristallite zur Folge haben. Sehr kleine Metallkristallite erhält man nur bei sehr geringen Metallmengen auf dem Träger. Die so erhaltene spezifische Metalloberfläche bleibt deshalb meist unter der von Katalysatoren mit hoher Beladung und großen Kristallen zurück.

Ein Verfahren, auch noch bei Beladungen von beispielsweise 25 Gew.% MeO (wobei Me für ein Eisenmetall oder Kupfer steht) auf einem Träger Kristallitgrößen im Bereich < 2,5 nm zu erhalten, ist die von Geus beschriebene Hydrolysefällung (Prep. Catal. III, 1 (1983)). Ausgangspunkt ist dabei ein in einer Lösung aus Metallnitrat und beispielsweise Harnstoff suspendierter pulverförmiger Träger. Durch thermische Zersetzung des Harnstoffs wird das Metall sehr homogen auf dem Träger niedergeschlagen und bildet bei der Reduktion sehr kleine Kristallite. Auf diese Weise ist es möglich, spezifische Metalloberflächen zu erhalten, wie sie sonst nur mit hoher Metallbeladung möglich sind.

Der so erhaltene pulverförmige Katalysator hat den Nachteil, daß er nicht ohne große Mengen an anorganischen Bindemitteln zu Formkörpern verarbeitet werden kann. Dadurch wird aber der Anteil der aktiven Komponente stark verringert, was zu deutlichen Einbußen in der Aktivität führt.

Man hat ferner bereits versucht, geformte Metallkatalysatoren mit hoher Metalldispersion herzustellen, und zwar durch eine Fällung nach der Hydrolysemethode auf vorgeformten Trägern. Untersuchungen hierzu wurden bereits von K.F. de Jong (Prep. Catal. V, 385 (1990)) vorgenommen, allerdings mit Mn und Mo. In einer sehr verdünnten Lösung der Metallnitrate und Harnstoff wurden SiO₂-Kugeln (Durchmesser bis 1,5 mm) eingebracht, und die Metalle wurden durch Zersetzung des Harnstoffs darauf abgeschieden. Der Nachteil dieses Verfahrens besteht darin, daß nur sehr geringe Metallbeladungen (bis 5 %) erreicht werden. Bei höheren Konzentrationen fällt ein Teil des Metalls in der Lösung als Me(OH)₂ aus.

In der US-A-3668148 wird dieses Verfahren zur Abscheidung von Nickel beschrieben, allerdings mit dem Nachteil, daß die Hälfte des eingesetzten Nickels in Lösung bleibt.

Aus der SU-A-459 247 ist ein Verfahren zur Herstellung von Nickelkatalysatoren für die Hydrierung von organischen Verbindungen sowie für die Feingasreinigung bekannt, bei der eine Fällung des Nickels aus Lösungen seiner Salze auf suspendiertem Kieselgel mittels einer wäßrigen Ammoniumhydroxid- oder Ammoniumcarbonatlösung durchgeführt wird. Die Fällung wird bei 70 bis 75°C und einem pH-Wert von 5 bis 7,5 durchgeführt. Der Niederschlag wird in der Mutterlauge 1,5 bis 2 Stunden bei 90 bis 95°C gealtert.

Nach dem Abfiltrieren, Waschen und Trocknen wird der Niederschlag calciniert und im Wasserstoffstrom reduziert. Die Katalysatoren haben einen Nickelgehalt von 10 bis 50 Gew.-%; die BET-Oberfläche des Trägers liegt zwischen 55 und 300 m²/g, die Nickeloberfläche zwischen 18 und 28 m²/g Katalysator. Obwohl angegeben ist, daß die hergestellten Katalysatoren Tabletten im Format 5 x 5 mm sind, finden sich keine Angaben über die Festigkeit dieser Tabletten bzw. wie Katalysatorkörper mit erhöhter Festigkeit erhalten werden können. Ferner finden sich keine Angaben über die Nickelkristallitgröße und das Porenvolumen.

Aus der FR-A-2 523 869 ist ein Oligomerisierungskatalysator auf der Basis von einwertigem Nickel auf einem Kieselsäureträger bekannt, der dadurch hergestellt wird, daß man den Kieselsäureträger mit einer wäßrigen Alkali- oder Ammoniumsalzlösung mit einem pH-Wert von mehr als etwa 10 behandelt, die so behandelte Kieselsäure mit einer wäßrigen Nickel(II)Salzlösung bei einem pH-Wert von etwa 8 behandelt, um die durch die Kieselsäure gebundenen Alkali- bzw. Ammoniumionen durch Nickelionen zu ersetzen, das erhaltene Produkt auf 300 bis 900°C erhitzt und das zweiwertige Nickel zu einwertigem Nickel reduziert. Das Nickel liegt also nicht als Metall vor, so daß über die Metall-Kristallitgröße keine Angaben gemacht werden können.

Aus der US-A-4 716 256 ist ein Verfahren zur selektiven Hydrierung von Diolefinen zu Monoolefinen bekannt, wobei ein Katalysator, der im wesentlichen aus elementarem Nickel auf einem anorganischen Träger besteht, verwendet wird. Der Katalysator kann 1 bis 40, vorzugsweise 2 bis 20 Gew.-% metallisches Nickel enthalten, wobei 5 bis 10 Gew.-% bevorzugt werden. Über die sonstigen Eigenschaften des Katalysators finden sich keine Angaben.

Aus der EP-B-0 307 520 ist ein Hydrierkatalysator, bestehend aus einem Aluminiumoxid-Trägermaterial, 0,05 bis 1,5 Gew.-% Schwefel und 1,0 bis 25 Gew.-% Nickel bekannt. Das Aluminiumoxid-Trägermaterial hat ein Porenvolumen von 1 bis 3 cm³/g und eine Oberfläche von größer als 150 m²/g, wobei weniger als 25 % des gesamten Porenvolumens von Poren gebildet werden, deren Porendurchmesser weniger als 15 nm beträgt und mehr als 60 % des Porenvolumens von Poren gebildet werden, deren Porendurchmesser mehr als 60 nm beträgt. Der Katalysator kann in Form von Pellets, Kugeln, Extrudaten oder unregelmäßig geformten Körpern vorliegen, doch finden sich keine Angaben über deren Festigkeit. Ferner finden sich keine Angaben über die Nickel-Kristallitgröße.

Aus der US-A-3 668 148 ist ein Verfahren zur Herstellung von Metallkatalysatoren auf einem teilchenförmigen Träger bekannt, wobei die Trägerteilchen in einer wäßrigen Lösung eines Salzes des Katalysatormetalls und einer Substanz, die beim Erhitzen in wäßriger Lösung Hydroxylionen freisetzt, suspendiert sind. Die Suspension wird in einem Autoklaven bei mehr als 100°C erhitzt, wobei sich die Teilchen des Katalysatormetalls gleichmäßig auf den Trägerteilchen abscheiden. Der Katalysator wird in der üblichen Weise reduziert. Es ist eine Vielzahl von Katalysatormetallen angegeben, wobei auch Metalle der Eisengruppe und Kupfer genannt sind. Über das Porenvolumen und die Bildung von Formkörpern finden sich keine Angaben.

Die Aufgabe der vorliegenden Erfindung bestand darin, hohe Metallbeladungen ohne jeglichen Verlust an Metall auf porösen Formkörpern zu erhalten, die auch ohne Bindemittel eine ausreichende Festigkeit bei gleichzeitig hohem spezifischem Porenvolumen haben.

Diese Aufgabe wird erfindungsgemäß mit Hilfe eines Katalysator-Formkörpers gelöst, der im reduzierten Zustand ein Metall der Eisengruppe und/oder Kupfer auf einem Träger enthält und der durch folgende Merkmale gekennzeichnet ist:
(a) Metallgehalt: 5-40 Gew.%, bezogen auf das Gesamtgewicht des Katalysators.
(b) Metall-Kristallitgröße ≦ 3nm
(c) Volumen der Poren mit einem Durchmesser von 7,5nm bis 15µm: 0,05 bis 0,9 ml/g Katalysator;
(d) BET-Oberfläche: 80-400 m²/g Katalysator;
(e) Bruchfestigkeit > 30 N (bezogen auf einen zylindrischen Formkörper mit einem Durchmesser von 1,5mm und einer Länge von 5 mm);
(f) Schüttgewicht: 250-350g/l.

Die Bestimmung der Metall-Kristallitgröße (Merkmal (b)) erfolgt über die Messung des intensivsten Röntgenbeugungsreflexes des Metalls, z.B. des Nickels, im reduzierten Zustand. Für die Messungen im Rahmen der vorliegenden Erfindung wurde ein Philips-Röntgendiffraktometer vom Typ PW 1729 verwendet. Es können natürlich auch andere Röntgendiffraktometer verwendet werden. Die Auswertung des intensivsten Reflexes erfolgte mit Hilfe der Voigt-Funktion, wie es bei J.I. Langford, J. Applied Crystall. 11, 10 (1978) beschrieben ist. Es handelt sich hierbei im wesentlichen um die Bestimmung der Halbwertsbreite des intensivsten Reflexes.

Das spezifische Porenvolumen wird nach der Methode der Quecksilberpenetration bestimmt, die bei J. van Brakel et al., Powder Technology 29 (1981),1 beschrieben ist. Bei dieser Methode wird das Quecksilber bis zu einem Druck von etwa 2000 bar in die Katalysator-Formkörper eingepreßt, wobei die Volumenabnahme des Quecksilbers als Funktion des Druckes aufgetragen wird. Auf diese Weise erhält man eine Kurve, aus deren Verlauf sich auch die Porenverteilung ermitteln läßt. Nach der Quecksilberpenetrationsmethode kann nur das Volumen und die Verteilung der Poren mit einem Durchmesser von > 7,5 nm bestimmt werden. Will man das Gesamtporenvolumen bestimmen, so wendet man die Methode der Wasseraufnahme nach DIN 51056 an. Nach dieser Methode wird eine genau abgewogene Menge der Katalysator-Formkörper mit Wasser von Umgebungstemperatur überschichtet, das mindestens 30 Min. am Kochen gehalten wird. Das überschüssige Wasser wird nach 4 h von den Formkörpern abfiltriert, und diese werden zurückgewogen. Die prozentuale Gewichtszunahme entspricht der Wasseraufnahme, woraus sich das Gesamt-Porenvolumen errechnen läßt.

Die BET-Oberfläche (Merkmal d) wird über die N₂-Sorption nach der Einpunkt-Methode bestimmt, wie es in DIN 66 132 angegeben ist.

Die Bruchfestigkeit (Merkmal e) wird unter Verwendung des Tablettenprüfgeräts 4M der Firma Schleuniger bestimmt. Der zylindrische Formkörper mit einer Länge von 5 mm wird zwischen die Backen der Vorrichtung gelegt, wobei ein Druck senkrecht zur Zylinderachse ausgeübt wird. Die Kraftsteigerungsrate beträgt 20 N/sec. Die Druckfestigkeit ergibt sich aus dem Mittel von 100 Messungen.

Das Schüttgewicht (Merkmal f) wird bestimmt, indem man einen Meßzylinder mit einem Durchmesser von 8 cm mit den zu untersuchenden Katalysator-Formkörpern bis zur 1-Liter-Marke füllt und das Gewicht der Formkörper bestimmt.

Bevorzugte Merkmale des erfindungsgemäßen Katalysators sind in den Unteransprüchen 2 bis 4 angegeben.

Die erfindungsgemäßen Katalysatoren-Formkörper zeichnen sich durch eine hohe Metallbeladung aus, ohne daß sich bei ihrer Herstellung die unlöslichen Metallverbindungen gesondert von den Trägerkörpern in der wässrigen Phase abscheiden.

Die erfindungsgemäßen Katalysator-Formkörper sind im wesentlichen nach zwei Varianten erhältlich. Nach der ersten Variante geht man so vor, daß man eine Verbindung, die sich beim Erhitzen in Lösung unter Erhöhung des pH-Wertes der Lösung langsam zersetzt, in Gegenwart eines pulverförmigen suspendierten Trägers auf eine Lösung eines Salzes eines Metalls der Eisengruppe und/oder des Kupfers einwirken läßt, um einen Niederschlag aus unlöslichen Metallverbindungen auf dem Träger zu erzeugen, den Niederschlag zusammen mit dem Träger von der Lösung abtrennt und noch im feuchten Zustand unter Wärmezufuhr plastifiziert und verformt, trocknet und/oder calciniert und die Metallverbindungen bzw. die getrockneten und/oder calcinierten Metallverbindungen reduziert.

Nach der zweiten Variante geht man so vor, daß man eine Verbindung, die sich beim Erhitzen in Lösung unter Erhöhung des pH-Wertes langsam zersetzt, in den Poren eines porösen Träger-Formkörpers auf eine Lösung eines Salzes eines Metalls der Eisengruppe und/oder des Kupfers einwirken läßt, um in den Poren des Träger-Formkörpers einen Niederschlag aus unlöslichen Metallverbindungen zu erzeugen, den Niederschlag zusammen mit dem Träger-Formkörper von der Lösung abtrennt, trocknet und/oder calciniert und die Metallverbindungen bzw. die getrockneten und/oder calcinierten Metallverbindungen reduziert.

Als Verbindungen, die sich beim Erhitzen in Lösung unter Erhöhung des pH-Wertes langsam zersetzen, verwendet man vorzugsweise Ammoniumcarbonat oder-bicarbonat; Harnstoff; ein Amid, wie Formamid, Dimethylformamid, Acetamid, Dimethylacetamid oder ein Amin, wie Hexamethylentetramin.

Die Metallsalzlösung wird vorzugsweise als konzentrierte Lösung eingesetzt, wobei nach der zweiten Verfahrensvariante ein hochporöser Träger mit einer Wasseraufnahme von mindestens 140 % verwendet wird. Bei beiden Verfahrensvarianten wird das Gemisch mit einer Verbindung, die sich beim Erhitzen in Lösung unter Erhöhung des pH-Wertes der Lösung langsam zersetzt, umgesetzt und anschließend bei einer Temperatur, die über der Zersetzungstemperatur dieser Verbindung liegt, im allgemeinen mehrere Stunden reagieren gelassen. Die sich beim Erhitzen unter Erhöhung des pH-Wertes der Lösung langsam zersetzende Verbindung wird vorzugsweise in einem 1,5-3 molaren Überschuß verwendet. Nach dem Ausfällen der Metallverbindung liegt deren Gehalt, berechnet als MeO, zwischen 5 und 40 Gew.%, während die Menge des Trägers 60 und 95 Gew.% beträgt.

Das Lösungsmittel hat vorzugsweise einen Siedepunkt, der höher ist als die Zersetzungstemperatur der sich unter Erhöhung des pH-Wertes zersetzenden Verbindung. In dem Lösungsmittel sind sowohl diese Verbindung als auch das Metallsalz löslich. Wird nach einer bevorzugten Ausführungsform der Erfindung Harnstoff verwendet, kommen als geeignete Lösungsmittel z. B. Wasser und/oder Ethylenglykol in Frage. Die Fällung wird eingeleitet durch die Zersetzung der sich unter Erhöhung des pH-Wertes zersetzenden Verbindung bei erhöhter Temperatur und geschieht über einen längeren Zeitraum, vorzugsweise über einen Zeitraum von 4-60 Stunden. Danach wird der Feststoff abfiltriert, gewaschen und vorzugsweise in ein beheizbares Mischaggregat überführt, wo er unter gleichzeitiger Plastifizierung auf die zur Verformung notwendige Feuchte, bevorzugt auf einen Trockenverlust zwischen 50 und 70 % gebracht wird. Die so erhaltene Paste wird zu Formkörpern verarbeitet, beispielsweise durch Aufbauagglomeration oder durch Extrudieren. Die Katalysatoren werden beispielsweise in die Form von gepressten Zylindern, Tabletten, Pastillen, Wagenrädern, Ringen, Sternen oder Strangpreßlingen, wie Vollsträngen, polylobären Strängen, Hohlsträngen und Wabenkörpern gebracht.

Nach einer besonders bevorzugten Ausführungsform der Erfindung wird die Paste zu Strängen mit einem Durchmesser von ≧ 1,5mm extrudiert und danach getrocknet. Vorzugsweise erfolgt die Trocknung zunächst bei einer Temperatur von ≧ 95 °C über einen Zeitraum von ≧ 4h. Die getrockneten Formkörper werden entweder sofort reduziert oder zuvor calciniert, wobei die Calcinierung bei einer Temperatur von ≧ 250 °C, vorzugsweise bei 300-500°C, vorzugsweise über einen Zeitraum von 2-10 h erfolgt.

Die Reduktion erfolgt vorzugsweise bei einer Temperatur zwischen 350 und 650°C, insbesondere bei 400-450°C und einer H₂ Belastung von 1-60 l/g Katalysator und Stunde. In den reduzierten Formkörpern liegen die Metallkristallitgrößen in einem Bereich von ≦ 3nm. Die Bruchfestigkeit der reduzierten und luftstabilisierten Formkörper liegt bei > 30 N, das spezifische Porenvolumen zwischen 0,05 und 0,9 ml/g und die BET-Oberfläche zwischen 80 und 400m²/g.

Nach der zweiten Ausführungsform der Erfindung wird ein hochporöser Träger mit einer Lösung aus Metallsalz und einem Überschuß der Verbindung, die sich beim Erhitzen in wäßrigem Medium unter Erhöhung des pH-Wertes langsam zersetzt, vorzugsweise in einem 1,5 bis 3-molaren Überschuß imprägniert, wobei die Konzentration des Metalls in der Lösung so berechnet wird, daß in dem Teil der Lösung, der die Poren der Formkörper ausfüllt, eine Metallmenge enthalten ist, die bezogen auf MeO, 5 bis 30 Gew.% des fertigen Katalysators ausmacht, wobei der Trägeranteil bei 70 bis 95 Gew.% liegt.

Für beide Ausführungsformen kommen alle Lösungsmittel in Betracht, deren Siedepunkt höher liegt als die Zersetzungstemperatur der Verbindung, die sich beim Erhitzen in Lösung unter Erhöhung des pH-Wertes langsam zersetzt. Sowohl diese Verbindung als auch das Metallsalz sind in dem Lösungsmittel in den vorstehend beschriebenen Mengen löslich. Wird bei einer besonders bevorzugten Ausführungsform der Erfindung Harnstoff verwendet, so kommen als geeignete Lösungsmittel z.B. Wasser und Ethylenglykol in Frage.

Nach einer besonders bevorzugten Ausführungsform der Erfindung werden die Formkörper mit der vorstehend beschriebenen Lösung überschichtet und längere Zeit, vorzugsweise mehr als 10 min stehengelassen, und anschließend von der überstehenden Lösung abgetrennt.

Danach werden die feuchten Formkörper in ein offenes Gefäß mit Rückflußkühler oder in ein geschlossenes Gefäß überführt und über einen längeren Zeitraum, vorzugsweise über einen Zeitraum von 8 - 60 h bei einer Temperatur gehalten, die höher ist als die Zersetzungstemperatur der Verbindung, die sich beim Erhitzen in Lösung unter Erhöhung des pH-Wertes langsam zersetzt. Während dieser Zeit zersetzt sich diese Verbindung und es fällt dabei das Metall als schwerlösliche Verbindung aus. Die so erhaltenen Formkörper werden gewaschen und anschließend getrocknet.

Die getrockneten Formkörper werden entweder sofort reduziert oder zuvor calciniert. Die Calcinierung erfolgt im allgemeinen bei Temperaturen von mehr als 250°C, vorzugsweise bei 300 - 500°C über einen Zeitraum von 2-10 h. Die Reduktion erfolgt vorzugsweise bei einer Temperatur zwischen 350 und 650 °C, besonders bevorzugt bei 400 - 450 °C und einer H₂-Belastung von 1 - 60 l pro g Katalysator und Stunde.

Gegenstand der Erfindung ist ferner die Verwendung der vorstehend beschriebenen Katalysator-Formkörper für die Hydrierung von ein- oder mehrfach ungesättigten organischen Verbindungen, insbesondere zur Hydrierung von Aromaten.

Gegenstand der Erfindung ist ferner die Verwendung der vorstehend beschriebenen Katalysator-Formkörper für Methanisierungen und Oligomerisierungen.

Gegenstand der Erfindung ist schließlich die nicht-reduzierte Vorstufe des vorstehend beschriebenen Katalysator-Formkörpers.

Die Erfindung ist durch die nachstehenden Beispiele in nicht einschränkender Weise erläutert.

### Beispiel 1

Eine Suspension aus 1 946,6 g Ni(NO₃)₂ x 6H₂O, 1 225,2 g Harnstoff und 1 500 g pyrogener Kieselsäure (BET-Oberfläche 150 m²/g) in 40 l Wasser wird unter Rühren bei 100°C unter Rückflußbedingungen reagieren gelassen, bis der Ni-Gehalt des Filtrats < 10 ppm ist, und danach abfiltriert. Der Filterkuchen wird dreimal mit heißem Wasser gewaschen und dann in einen beheizten Kneter überführt, wo er unter ständigem Knetem bis auf Extrudierfeuchte (etwa 56 Gew.% Wasser) gebracht wird. Die Masse wird zu Strängen mit einem Durchmesser von 2 mm extrudiert. Die feuchten Extrudate schrumpfen beim Trocknen auf 1,5 mm Durchmesser. Sie haben nach der Calcinierung bei 400°C folgende Eigenschaften:

| | |
|---|---|
| NiO-Gehalt | 25 Gew.% |
| BET-Oberfläche | 199 m²/g |
| spez. Porenvolumen | 0,83 ml/g |
| Bruchfestigkeit | 37 N |

Die getrockneten Extrudate werden bei 400°C im Wasserstoffstrom reduziert. Man erhält Ni-Kristallite, deren Größe unterhalb der kleinsten, aus Röntgenbeugungsmeßungen zu berechnenden Größe von 2,8 nm liegt.

### Beispiel 2

Eine Suspension aus 285 g Cu(NO₃)₂ x 3H₂O, 211,8 g Harnstoff und 175 g pyrogener Kieselsäure (BET-Oberfläche 150 m²/g) in 5 l Wasser wird unter Rühren bei 100°C unter Rückflußbedingungen reagieren gelassen, bis der Cu-Gehalt des Filtrats < 10 ppm ist, und danach abfiltriert. Der Filterkuchen wird dreimal mit heißem Wasser gewaschen und dann in einen beheizten Kneter überführt, wo er unter ständigem Kneten bis auf Extrudierfeuchte (etwa 56 Gew.% Wasser) gebracht wird. Die Masse wird zu Strängen mit einem Durchmesser von 2 mm extrudiert. Die feuchten Extrudate schrumpfen beim Trocknen auf 1,5 mm Durchmesser. Sie haben nach der Calcinierung bei 450 °C folgende Eigenschaften:

| | |
|---|---|
| CuO-Gehalt | 37,5 Gew.% |
| BET-Oberfläche | 390 m²/g |
| Spez. Porenvolumen | 0,72 ml/g |
| Bruchfestigkeit | 35 N. |

Die getrockneten Extrudate werden bei 400°C im Wasserstoffstrom reduziert. Man erhält Cu-Kristallite mit einer Größe von ≦ 3,0 nm.

### Beispiel 3

Eine Suspension aus 295 g Fe(NO₃)₃ x 9H₂O, 134 g Harnstoff und 175 g pyrogener Kieselsäure (BET-Oberfläche 150 m²/g) in 5 l Wasser wird bei 100 °C unter Rückflußbedingungen reagieren gelassen, bis der Fe-Gehalt des Filtrats < 10 ppm ist, und danach abfiltriert. Der Filterkuchen wird dreimal mit heißem Wasser gewaschen und dann in einen beheizten Kneter überführt, wo er unter ständigem Kneten bis auf Extrudierfeuchte (etwa 56 Gew.%Wasser) gebracht wird. Die Masse wird zu Strängen von 2 mm Durchmesser extrudiert. Die feuchten Extrudate schrumpfen beim Trocknen auf 1,5 mm Durchmesser. Sie haben nach der Calzinierung bei 400 °C folgende Eigenschaften:

| | |
|---|---|
| Fe₂O₃-Gehalt | 25 Gew.% |
| BET-Oberfläche | 108 m²/g |
| spez. Porenvolumen | 0,86 ml/g |
| Bruchfestigkeit | 33 N |

Die getrockneten Extrudate werden bei 400°C im Wasserstoffstrom reduziert. Man erhält Fe-Kristallite, deren Größe unterhalb der kleinsten, aus Röntgenbeugungsmessungen zu berechnenden Größe von 2,8 nm liegt.

### Beispiel 4

Eine Suspension aus 226,6 g Co(NO₃)₂ x 6H₂, 140 g Harnstoff und 175 g pyrogener Kieselsäure (BET-Oberfläche 150 m²/g) in 5 l Wsser wird unter Rühren bei 100 °C unter Rückflußbedingungen reagieren gelassen, bis der Co-Gehalt des Hydrats < 10 ppm ist, und danach abfiltriert. Der Filterkuchen wird dreimal mit heißem Wasser gewaschen und dann in einen beheizten Kneter überführt, wo er unter ständigem Kneten bis auf Extrudierfeuchte (etwa 56 Gew. % H₂O) gebracht wird. Die Masse wird zu Strängen von 2 mm Durchmesser extrudiert. Die feuchten Extrudate schrumpfen beim Trocknen auf 1.5 mm Durchmesser. Sie haben nach der Calzinierung bei 400 °C folgende Eigenschaften:

| | |
|---|---|
| CoO-Gehalt | 25 Gew. % |
| BET-Oberfläche | 238 m²/g |
| spez. Porenvolumen | 0,64 ml/g |
| Bruchfestigkeit | 36 N |

Die getrockneten Extrudate werden bei 400 °C im Wasserstoffstrom reduziert. Man erhält Co-Kristallite, deren Größe unterhalb der kleinsten, aus Röntgenbeugungsmessungen zu berechnenden Größe von 2,8 nm liegt.

### Beispiel 5

259,5 g Ni(NO₃)₂ x 6H₂O und 107,2 g Harnstoff werden in soviel Wasser gelöst, daß ein Lösungsvolumen von 290 ml entsteht. 100 g SiO₂-Extrudate (Durchmesser 1,5 mm) mit einer Wasseraufnahme von 145 % werden mit dieser Lösung überschichtet, 1 h stehengelassen und dann abfiltriert. Die imprägnierten Extrudate werden in einen Kolben mit Rückflußkühler überführt, im Ölbad auf 90 °C erhitzt und 24 h bei dieser Temperatur gehalten. Danach werden sie dreimal mit heißem Wasser gewaschen, getrocknet und 4 h bei 400 °C calciniert. Sie haben dann folgende Eigenschaften:

| | |
|---|---|
| NiO-Gehalt | 24,2 Gew.% |
| BET-Oberfläche | 242 m²/g |
| spez. Porenvolumen | 0,70 ml/g |
| Bruchfestigkeit | 41 N |

Die getrockneten Extrudate werden bei 400°C im Wasserstoffstrom reduziert. Man erhält Ni-Kristallite, deren Größe unterhalb der kleinsten, aus Röntgenbeugungsmessungen zu berechnenden Größe von 2,8 nm liegt.

### Beispiel 6 (Vergleich)

Die in Beispiel 5 erwähnten Extrudate werden mit der dort beschriebenen Nickelnitratlösung, allerdings ohne Harnstoffzusatz, imprägniert und im Trockenschrank 24 h bei 120°C getrocknet und 4 h bei 400°C calciniert. Sie haben dann folgende Eigenschaften:

| | |
|---|---|
| NiO-Gehalt | 25,2 Gew.% |
| BET-Oberfläche | 235 m²/g |
| spez. Porenvolumen | 0,68 ml/g |
| Bruchfestigkeit | 31 N |

Die getrockneten Extrudate werden bei 400°C im Wasserstoffstrom reduziert. Man erhält Ni-Kristallite mit einer Größe von Die getrockneten Extrudate werden bei 400°C im Wasserstoffstrom reduziert. Man erhält Ni-Kristallite mit einer Größe von 9,8 nm.

### Beispiel 7 (Anwendung)

Zur Durchführung einer Hydrierung von Toluol zu Methylcyclohexan in der Tricklephase (Rieselphase) werden 15 ml des nach Beispiel 5 hergestellten Katalysators, verdünnt mit Glaskugeln, in einen Rohrreaktor überführt. Die Reaktion läuft bei 30 bar und 110°C über 70 h. Als Einsatzmaterial wird eine Mischung aus 30 Gew.% Toluol und 70 Gew.% Methylcyclohexan verwendet. Die Belastung (LHSV = Liquid Hourly Space Velocity) beträgt 1 Liter (Toluol) /1 Liter (Kat) x h, der Wasserstoffdurchsatz 1 450 Liter/Liter Toluol. Der mittlere Umsatz an Toluol beträgt dabei 58,5 %.

### Beispiel 8 (Anwendung /Vergleich)

Der nach Beispiel 6 hergestellte Katalysator wird unter den in Beispiel 7 genannten Bedingungen getestet. Der mittlere Umsatz an Toluol beträgt dabei 42,3 %.

Wie der Vergleich der Beispiele 5 und 6 bzw. 7 und 8 zeigt, liegt der Vorteil des erfindungsgemäßen Katalysators in einer um 30 % höheren Festigkeit und wesentlich kleineren Kristallen, als bei dem Vergleichskatalysator. Dieses letzere Merkmal bewirkt einen um fast 40 % höheren Umsatz mit dem erfindungsgemäßen Katalysator an Toluol in der Testreaktion, im Verhältnis zum Vergleichskatalysator.

## Patentansprüche

1. Katalysator-Formkörper, der im reduzierten Zustand ein Metall der Eisengruppe und/oder Kupfer auf einem Träger enthält, gekennzeichnet durch folgende Merkmale:
(a) Metallgehalt: 5-40 Gew.%, bezogen auf das Gesamtgewicht des Katalysators.
(b) Metall-Kristallitgröße ≦ 3nm
(c) Volumen der Poren mit einem Durchmesser von 7,5nm bis 15µm: 0,05 bis 0,9 ml/g Katalysator;
(d) BET-Oberfläche: 80-400 m²/g Katalysator;
(e) Bruchfestigkeit > 30 N (bezogen auf einen zylindrischen Formkörper mit einem Durchmesser von 1,5mm und einer Länge von 5mm);
(f) Schüttgewicht: 250-350g/l.

2. Katalysator-Formkörper nach Anspruch 1, dadurch gekennzeichnet, daß der Träger aus einem pulverförmigen Oxid oder Silicat hergestellt ist.

3. Katalysator-Formkörper nach Anspruch 2, dadurch gekennzeichnet, daß der Träger aus pulverförmiger pyrogener Kieselsäure hergestellt ist.

4. Katalysator-Formkörper nach einem der Ansprüche 1 bis 3, gekennzeichnet durch folgende Porendurchmesserverteilung:
7,5 nm - 14 nm: 0 bis 2 %
14 nm - 80 nm: 1 bis 25 %
80 nm - 1,75µm: 50 bis 90 %
1,75µm - 15 µm: 0 bis 15 %

5. Katalysator-Formkörper, der im reduzierten Zustand ein Metall der Eisengruppe und/oder Kupfer auf einem Träger enthält, insbesondere nach einem der Ansprüche 1 bis 4, dadurch erhältlich, daß man eine Verbindung, die sich beim Erhitzen in Lösung unter Erhöhung des pH-Wertes des wässrigen Mediums langsam zersetzt, in Gegenwart eines pulverförmigen suspendieren Trägers auf eine Lösung eines Salzes eines Metalls der Eisengruppe und/oder des Kupfers einwirken läßt, um einen Niederschlag aus unlöslichen Metallverbindungen auf dem Träger zu erzeugen, den Niederschlag zusammen mit dem Träger von der Lösung abtrennt und noch im feuchten Zustand unter Wärmezufuhr plastifiziert und verformt, trocknet und/oder calciniert und die Metallverbindungen bzw. die getrockneten und/oder calcinierten Metallverbindungen reduziert.

6. Katalysator-Formkörper, der im reduzierten Zustand ein Metall der Eisengruppe und/oder Kupfer auf einem Träger enthält, insbesondere nach einem der Ansprüche 1 bis 4, dadurch erhältlich, daß man eine Verbindung, die sich beim Erhitzen in Lösung unter Erhöhung des pH-Wertes langsam zersetzt, in den Poren eines porösen Träger-Formkörpers auf eine Lösung eines Salzes eines Metalls der Eisengruppe und/oder des Kupfers einwirken läßt, um in den Poren des Träger-Formkörpers einen Niederschlag aus unlöslichen Metallverbindungen zu erzeugen, den Niederschlag zusammen mit dem Träger-Formkörper von der Lösung abtrennt, trocknet und/oder calciniert und die Metallverbindungen bzw. die getrockneten und/oder calcinierten Metallverbindungen reduziert.

7. Katalysator-Formkörper nach Anspruch 5 oder 6, dadurch gekennzeichnet, daß die Verbindung, die sich beim Erhitzen in Lösung unter Erhöhung des pH-Wertes langsam zersetzt, Ammoniumcarbonat oder-bicarbonat; Harnstoff; ein Amid, wie Formamid, Dimethylformamid, Acetamid, Dimethylacetamid; oder ein Amin, wie Hexamethylentetramin, darstellt.

8. Katalysator-Formkörper nach einem der Ansprüche 1 bis 7, dadurch erhältlich, daß die Verbindung, die sich beim Erhitzen in Lösung unter Erhöhung des pH-Wertes zersetzt, bei einer Temperatur von > 50°C, vorzugsweise von > 90°C zersetzt wird, wobei sich der Formkörper in einer gesättigten Lösungsmitteldampf-Atmosphäre befindet.

9. Katalysator-Formkörper nach einem der Ansprüche 1 bis 8, dadurch erhältlich, daß das Lösungsmittel einen Siedepunkt hat, der höher ist als die Zersetzungstemperatur der sich unter Erhöhung des pH-Wertes zersetzenden Verbindung.

10. Katalysator-Formkörper nach Anspruch 5, dadurch gekennzeichnet, daß die Wärmezufuhr in einem beheizten Mischaggregat erfolgt.

11. Katalysator-Formkörper nach Anspruch 5, erhältlich durch Aufbauagglomeration oder Extrudieren.

12. Katalysator-Formkörper nach einem der Ansprüche 1 bis 4 und 6 bis 9, erhältlich durch Imprägnieren des Trägers mit überschüssiger Lösung.

13. Katalysator-Formkörper nach einem der Ansprüche 1 bis 4, und 6 bis 9, dadurch gekennzeichnet, daß die Träger-Formkörper nach der Porenfüllungsmethode imprägniert sind, wobei das Volumen der Imprägnierlösung maximal dem Porenvolumen der Formkörper entspricht.

14. Katalysator-Formkörper nach einem der Ansprüche 1 bis 13, dadurch erhältlich, daß der feuchte Katalysator-Formkörper zunächst bei einer Temperatur von ≧ 95°C über einen Zeitraum von ≧ 4 h getrocknet und anschließend bei einer Temperatur von ≧ 250°C, vorzugsweise von 300-500°C calciniert wird.

15. Katalysator-Formkörper nach einem der Ansprüche 1 bis 14, in Form von gepreßten Zylindern, Tabletten, Pastillen, Wagenrädern, Ringen, Sternen oder Strangpreßlingen, wie Vollsträngen, polylobären Strängen, Hohlsträngen und Wabenkörpern.

16. Nicht-reduzierte Vorstufe des Katalysator-Formkörpers nach einem der Ansprüche 1 bis 15.

17. Verwendung der Katalysator-Formkörper nach einem der Ansprüche 1 bis 15 für die Hydrierung von ein- und mehrfach ungesättigten organischen Verbindungen, insbesondere zur Hydrierung von Aromaten.

18. Verwendung der Katalysator-Formkörper nach einem der Ansprüche 1 bis 15 für Methanisierungen.

19. Verwendung der Katalysator-Formkörper nach einem der Ansprüche 1 bis 15 für Oligomerisierungen.
